(19) Europäisches Patentamt
European Patent Office
Office européen des brevets

(11) Veröffentlichungsnummer : **0 029 936**
**B1**

(12) **EUROPÄISCHE PATENTSCHRIFT**

(45) Veröffentlichungstag der Patentschrift : **21.03.84**

(51) Int. Cl.³ : **G 01 N 31/06**, G 01 N 19/10, G 01 N 27/12

(21) Anmeldenummer : **80106931.1**

(22) Anmeldetag : **10.11.80**

(54) **Verfahren und Vorrichtung zur Langzeitüberwachung gasförmiger Schadstoffemissionen und -immissionen, sowie des Feuchtegehaltes von Gasen, insbesondere in Luft.**

(30) Priorität : **16.11.79 DE 2946402**
**16.11.79 DE 2946424**

(43) Veröffentlichungstag der Anmeldung : **10.06.81 Patentblatt 81/23**

(45) Bekanntmachung des Hinweises auf die Patenterteilung : **21.03.84 Patentblatt 84/12**

(84) Benannte Vertragsstaaten : **FR GB IT NL**

(56) Entgegenhaltungen :
**CA-A- 981 054**
**DE-A- 1 957 764**
**DE-A- 2 710 847**
**DE-B- 1 100 337**
**GB-A- 1 387 201**
**US-A- 3 674 439**
**US-A- 3 927 555**

(73) Patentinhaber : **SIEMENS AKTIENGESELLSCHAFT**
**Berlin und München Wittelsbacherplatz 2**
**D-8000 München 2 (DE)**

(72) Erfinder : **Pink, Hans, Dr.**
**Possenhofener Strasse 38a**
**D-8130 Starnberg (DE)**

**EP 0 029 936 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren und Vorrichtung zur Langzeitüberwachung gasförmiger Schadstoffemissionen und -immissionen, sowie des Feuchtegehaltes von Gasen, insbesondere in Luft

Die vorliegende Patentanmeldung betrifft ein Verfahren zur Langzeitüberwachung gasförmiger Schadstoffemissionen und -immissionen, sowie des Feuchtegehaltes von Gasen, insbesondere in Luft, bei dem als Detektionsprinzip die durch den Schadstoff oder die Feuchte als Reaktionsgas A mit einem festen Reaktionspartner B erfolgte chemische Reaktion oder Adsorption verwendet und quantitativ dadurch ausgewertet wird, daß zur quantitativen Auswertung die durch das Reaktionsgas A bewirkte Volumenänderung des Reaktionspartners B in eine äquivalente elektrische Größe übergeführt wird. Ferner betrifft die Patentanmeldung eine Vorrichtung zur Durchführung dieses Verfahrens mit einem Reaktor, bestehend aus einem mit einer Gaseinlaßöffnung und einer Absaugvorrichtung versehenen rohrförmigen Reaktionsraum.

Dieses Prinzip ist aus der DE-A1-2 710 847 und der US-A-3 927 555 zu entnehmen. Dabei wird die bei der Absorption bzw. chemischen Reaktion von $H_2$ mit einem wasserstoffabsorbierenden Metall oder einer Metallegeirung bewirkte Volumenänderung in eine äquivalente elektrische Größe wie den Widerstand eines Dehnungsmeßstreifens überführt oder der Wasserstoffpartialdruck über Längenänderungsmessungen eines Rohres aus einer Pd-Legierung mittels eines Differentialtrafos ermittelt.

Aus dem Prüfröhrchen-Taschenbuch von Dräger, 4. Ausgabe (Mai 1979), Sonderdruck 2340 ist ein Detektionsprinzip bekannt, bei dem die durch einen Schadstoff oder die Feuchte als Reaktionsgas A mit einem festen Reaktionspartner B erfolgte chemische Reaktion oder Adsorption verwendet und quantitativ ausgewertet wird. Das Prinzip der hier benutzten Prüfröhrchen (Dräger-Röhrchen genannt) beruht darauf, daß eine für das zu detektierende Gas spezifische chemische Verbindung als Füllstoff in das Röhrchen gebracht und mit dem angesaugten Gas beladen wird, wobei sich entsprechend der Gaskonzentration und der Einwirkungsdauer (durchgesaugte Luft bzw. Zahl der Hübe) eine verfärbte Zone der Füllstoffsäule oder eine durch Quellung verlängerte Zone der Füllstoffsäule ausbildet. Als Maß für den Schadstoffgehalt bzw. Feuchtegehalt wird dabei die Länge der verfärbten bzw. zum Quellen gebrachte Zone angesehen.

Mit dieser Methode können Gase und Dämpfe wie Schwefelwasserstoff, Schwefeldioxid, Schwefelkohlenstoff, Kohlenmonoxid, Kohlendioxid, Alkohol, Benzol, Kohlenwasserstoffe, Nitrosegase und Wasserdampf detektiert und zum Teil auch quantitativ erfaßt werden, wenn die genannten Stoffe eine spezifische Farbreaktion mit einem Festkörper eingehen. Ein Beispiel ist durch die Reaktion

$$H_2S + Pb^{++} \rightarrow PbS + 2\ H^+$$

gegeben. Dabei wird zur Untersuchung von Schwefelwasserstoff ($H_2S$) ein Prüfröhrchen verwendet, welches als Reagenz mit einem bleihaltigen Präparat, auf Kieselgel aufgetragen, gefüllt ist. Mit Hilfe von Eichpräparaten kann dann die Menge des zu Bleisulfid (PbS = verfärbte Zone) umgesetzten Schwefelwasserstoffes berechnet werden. Zur Untersuchung des Wassergehaltes von Luft wird beispielsweise ein Prüfröhrchen verwendet, welches als Reagens mit Stärke oder Kieselgel gefüllt ist.

Neben dem Nachteil der visuellen Messung besteht ein weiterer Nachteil dieser Methoden darin, daß eine Fernmessung und damit gekoppelte Zeitvorgaben, Begrenzungsschaltungen usw. nicht ohne größeren meßtechnischen Aufwand durchgeführt werden können.

Die Erfindung dient deshalb zur Lösung der Aufgabe, ein Verfahren bzw. eine Vorrichtung anzugeben, mit dem bzw. mit der es möglich ist, den Schadstoffgehalt bzw. den Feuchtegehalt in Gasen, insbesondere in Luft über längere Zeiträume unabhängig von einer visuellen Beobachtung kontinuierlich zu messen.

Diese Aufgabe wird durch ein Verfahren der eingangs genannten Art dadurch gelöst, daß erfindungsgemäß die Volumenänderung in eine äquivalente Druckänderung des Systems übergeführt wird und diese Druckänderung durch Messung des elektrischen Widerstandes von die Druckänderung anzeigenden Graphitteilchen, mittels einer dünnen Membran einer Piezokeramik oder über Drucksensoren auf Halbleiterbasis gemessen wird.

Die Graphitteilchen können aus einer kleinen Säule aus Graphitgrieß, Graphitkugeln oder aufeinander gestapelten Graphitscheibchen bestehen.

Eine Vorrichtung zur Durchführung des Verfahrens ist dadurch gekennzeichnet, daß an dem der Gaseinlaßöffnung entgegengesetzten Ende des Reaktionsraumes ein die Druckänderung anzeigendes System, bestehend aus einem Stapel von Graphitscheibchen angeordnet ist und einer elektrischen Widerstandsmessanordnung, deren Anschlüsse mit dem obersten und untersten Graphitscheibchen verbunden sind.

Wie für andere technische Anwendungen, wie z. B. Kohlegrieß oder Piezokeramik in der Sprechmuschel des Fernsprechers oder gestapelte Kohlescheibchen als Stromregler für Elektromotoren, sollen die Widerstandsänderungen der Kohleteilchen bzw. der piezoelektrische Effekt der Keramik hier zur Anzeige des sich ändernden Druckes und damit des Fortschreitens einer ablaufenden Reaktion benutzt werden. Die Selektivität der Anzeige für einen Stoff wird dabei erreicht durch die Auswahl einer chemischen Reaktion, die in einem Reaktor, z. B. in Form eines Röhrchens, stattfindet.

Zwei Beispiele für Volumenänderungen im Sinne der Erfindung sind die Oxidation von Eisen und die

Bildung von Bleisulfid aus Bleiacetat:

Nach der Gleichung

$$2\ Fe + 1{,}5\ O_2 \rightarrow Fe_2O_3$$

entstehen aus 112 g Fe, das ein Volumen von 14 $cm^3$ einnimmt, 160 g $Fe_2O_3$ mit einem Volumen von 30 $cm^3$ ($d_{Fe} = 7{,}86$, $d_{Fe_2O_3} = 5{,}18$), d. h. bei der Reaktion mit Sauerstoff verdoppelt eine gegebene Menge des Festkörpers Eisen sein Volumen.

Nach der Gleichung

$$Pb(CH_3COO)_2 + H_2S \rightarrow PbS + 2\ CH_3COOH$$

werden 325 g Bleiacetat mit einem Volumen von 100 $cm^3$ durch Schwefelwasserstoff zu 239,3 g Bleisulfid umgesetzt, das nur noch ein Volumen von 32 $cm^3$ einnimmt, d. h. bei dieser Reaktion tritt eine Verminderung des Volumens auf 1/3 seines Ausgangswertes ein, wenn die als Nebenprodukt der Reaktion entstehende Essigsäure ausgetrieben wird.

Läßt man nun eine derartige Reaktion in einem fest umschlossenen Reaktionsraum, z. B. einem Glasrohr mit Gasein- und -auslaß ablaufen, so verursacht das anwachsende Volumen eine zunehmende, das kleinerwerdende Volumen eine abnehmende Kraft auf die Gefäßwände.

Gemäß einem Ausführungsbeispiel nach der Lehre der Erfindung werden störende Komponenten (wenn erforderlich) durch eine Filterschicht absorbiert und abgetrennt.

Weitere Einzelheiten sind anhand von zwei Ausführungsbeispielen, nämlich der Reaktion von Kohlendioxid ($CO_2$) mit Natronasbest sowie der Adsorption (Quellung) von Wasserdampf aus Luft in Stärke bei der Beschreibung der in der Figur schematisch dargestellten Anordnung erläutert. Dabei wird als Reaktor 1 ein gerades, zur Verlängerung der druckaufbauenden Zone möglichst langes und dünnes Rohr aus Glas mit gekörntem Natronasbest 2 gefüllt. Anstelle von gekörntem Natronasbest wird bei Feuchtemessungen ein Adsorbens z. B. in Form von Stärke eingefüllt. Ist die eben beschriebene Reaktorform nicht möglich — weil z. B. das Volumen zur Aufnahme des Reaktionspartners zu klein ist — so kann die Druckänderung in einem größeren Volumen auch von einer in der Reaktorachse angeordneten, in einem flexiblen Schlauch befindlichen Flüssigkeit aufgenommen und hydraulisch auf das Meßsystem übertragen werden. Eine Kammer 3 zur Aufnahme von Filterstoffen muß vom Reaktionsraum durch eine starre Wand 4 getrennt sein, damit eine auch an dieser Stelle durch die auszufilternden Störstoffe entstehende Volumenänderung den Meßwert nicht verfälschen kann. Das zu überprüfende $CO_2$/Luft-Gemisch bzw. $H_2O$/Luft-Gemisch wird mit Hilfe z. B. einer kleinen Membranpumpe (siehe Pfeil 5) an der mit dem Pfeil 6 bezeichneten Stelle in den Reaktor 1 eingesaugt. Um zu verhindern, daß durch zeitweises Abschalten der Pumpe (5) ein zu geringer Wert der zu überwachenden Gasemission vorgetäuscht werden kann, wird der Betrieb der Pumpe (5) durch einen Betriebsstundenzähler (nicht dargestellt) überprüft. Ein solcher Betriebsstundenzähler kann beispielsweise aus einem, mit einer Kupfersalzlösung gefüllten Kapillarrohr bestehen, an dessen Innenwand sich eine dem fließenden Strom äquivalente Kupfermenge abscheidet, die ein Maß für die Betriebsdauer ist.

Am Ende der druckaufbauenden Zone des Reaktionsraumes 1 befindet sich als Drucksensor ein Stapel von 25 dünnen (0,25 mm) Graphitscheibchen 7 mit 9 mm ∅, wobei jeweils das erste und letzte Graphitscheibchen mit Platindrähten 8 und 9 kontaktiert ist, die isoliert aus dem Rohr herausgeführt sind. Gegen das Rohrende werden die Graphitscheibchen 7 durch eine Verschraubung 10, gegen den Reaktionsraum durch einen kleinen frei beweglichen Quarzklotz 11 gehaltert. Je eine eingeschmolzene Fritte (4 und 13) nach dem Gaseinlaß (6) und vor dem Gasauslaß (5) begrenzen das Probenvolumen gegenüber diesen beiden Öffnungen (5, 6).

Nach Füllen des Rohres 1 mit gekörntem Natronasbest 2 bzw. mit Stärke 2 wird durch Drehen der Schraubkappe 10 die Packungsdichte der Graphitscheibchen 7 willkürlich fest eingestellt. Der in diesem Zustand zwischen den Platindrähten 8 und 9 gemessene Widerstand wird als Ausgangswiderstand $R_0$ angesehen. Dann wird das getrocknete $CO_2$/Luft-Gemisch oder das zu messende $H_2O$/Luft-Gemisch durch das Rohr 1 geleitet und die dabei eintretende Widerstandsänderung gemessen.

Natronasbest besteht aus Asbestfasern mit eingelagertem Natriumhydroxid. Dieses reagiert gemäß

$$2\ NaOH + CO_2 \rightarrow Na_2CO_3 + H_2O$$

Dabei entsteht aus 2 Mol NaOH (= 80 g) mit 1 Mol $CO_2$ (= 44 g) 1 Mol $Na_2CO_3$ (= 106 g) und 1 Mol $H_2O$ (= 18 g). Unter Berückksichtigung des Dichteunterschiedes zwischen NaOH (2,130 g/$cm^3$) und $Na_2CO_3$ (2,532 g/$cm^3$) ändert sich das Volumen bei einem molaren Umsatz von 170,4 $cm^3$ auf 268,4 $cm^3$ (+ 18 $cm^3$ $H_2O$) entsprechend 68 % Volumenzunahme. Dieser Wert errechnet sich für reines Natriumhydroxid, für Natronasbest liegt er tiefer, wegen der « Verdünnung » durch die Asbestfaser.

## Ergebnis

Mit der erfindungsgemäßen Anordnung wurde eine Abnahme des vor der Reaktion eingestellten

Ausgangswiderstandes $R_0$ gemessen. Sie betrug in Parallelversuchen bei zweistündiger Reaktionszeit jeweils 2Ω. Dieser geringe Effekt ist darauf zurückzuführen, daß die elastischen Asbestfasern als Puffer wirken, indem sie die bei der Volumenvergrößerung sich aufbauende Kraft auffangen, so daß diese nur zum Teil zur Verdichtung des Graphitscheibchenstapels genutzt werden.

Anstelle der aufgestapelten Graphitscheibchen 7 kann auch eine Piezokeramik oder eine Halbleiteranordnung als Drucksensor verwendet werden.

Da der Reaktor 1, welcher jeweils spezifisch für einen Schadstoff (Schwefelwasserstoff, Kohlendioxid, Kohlenmonoxid, Kohlenwasserstoffe, Schwefeldioxid, Nitrosegase ($NO_x$), Arsenwasserstoff) mit den entsprechenden Reagenzien (Bleiverbindungen, Bariumhydroxid, Jodpentoxid, Selendioxid mit Thioschwefelsäure, Dinatriumtetrachloromercurat, Chrom (VI)-Verbindungen, Goldverbindungen) ausgestattet ist, in der Anordnung beliebig auswechselbar ist, können mit der Vorrichtung nach der Lehre der Erfindung eine Vielzahl von Gasen und Dämpfen erfaßt und in elektrische Meßdaten umgewandelt werden. Dadurch ergibt sich gegenüber der kompliziert aufgebauten, auf dem Effekt der Chromatographie beruhenden Drägerröhrchen die Möglichkeit, Fernmessungen, Zeitvorgaben, Begrenzungsschaltungen usw. durchzuführen.

Mit der Vorrichtung nach der Lehre der Erfindung können auch eine Vielzahl von feuchteenthaltenden Stoffen, insbesondere auch die in der Halbleitertechnologie verwendbaren Schutz- oder Trägergase auf ihren Feuchtegehalt erfaßt und in elektrische Meßdaten umgewandelt werden.

**Ansprüche**

1. Verfahren zur Langzeitüberwachung gasförmiger Schadstoffemissionen und -immissionen, sowie des Feuchtegehaltes von Gasen, insbesondere in Luft, bei dem als Detektionsprinzip die durch den Schadstoff oder die Feuchte als Reaktionsgas A mit einem festen Reaktionspartner B erfolgte chemische Reaktion oder Adsorption verwendet und quantitativ dadurch ausgewertet wird, daß zur quantitativen Auswertung die durch das Reaktionsgas A bewirkte Volumenänderung des Reaktionspartners B in eine äquivalente elektrische Größe übergeführt wird, dadurch gekennzeichnet, daß diese Volumenänderung in eine äquivalente Druckänderung des Systems übergeführt wird und diese Druckänderung durch Messung des elektrischen Widerstandes von die Druckänderung anzeigenden Graphitteilchen, mittels einer dünnen Membran einer Piezokeramik oder über Drucksensoren auf Halbleiterbasis gemessen wird.

2. Verfahren nach Anspruch 1, dadurch gekennzeichnet, daß die Graphitteilchen in Grießform, Kugelform oder als aufgestapelte Scheibchen vorliegen.

3. Verfahren nach einem der Ansprüche 1 und 2, dadurch gekennzeichnet, daß eine, die Reaktion oder Adsorption störenden Komponenten absorbierende Filterschicht vor den Reaktionsraum geschaltet wird.

4. Vorrichtung zur Durchführung des Verfahrens nach einem der Ansprüche 1 bis 3, mit einem Reaktor bestehend aus einem mit einer Gaseinlaßöffnung (6) und einer Absaugvorrichtung (5) versehenen rohrförmigen Reaktionsraum (1), dadurch gekennzeichnet, daß an dem der Gaseinlaßöffnung (6) entgegengesetztem Ende des Reaktionsraumes (1) ein die Druckänderung anszeigendes System, bestehend aus einem Stapel von Graphitscheibchen (7) angeordnet ist und einer elektrischen Widerstandsmeßanordnung, deren Anschlüsse (8, 9) mit dem obersten und untersten Graphitscheibchen (7) verbunden sind.

5. Vorrichtung nach Anspruch 4, dadurch gekennzeichnet, daß der Reaktionsraum (1) als gebogenes Rohr ausgebildet ist.

6. Vorrichtung nach Anspruch 4, dadurch gekennzeichnet, daß vor den Reaktionsraum (1) eine Filterschicht in einer, gegenüber dem Reaktionsraum (1) eine starre Wand (4) aufweisenden Kammer (3) angeordnet ist.

7. Vorrichtung nach einem der Ansprüche 4 bis 6, dadurch gekennzeichnet, daß anstelle des rohrförmigen Reaktionsraumes (1) ein in der Reaktorachse angeordneter, flexibler, mit Flüssigkeit gefüllter Schlauch angeordnet ist, wobei die Druckänderung über die im Schlauch befindliche Flüssigkeit aufgenommen und hydraulisch auf das Meßsystem übertragen wird.

8. Vorrichtung nach einem der Ansprüche 4 bis 7, dadurch gekennzeichnet, daß ein Betriebsstundenzähler in Form eines, eine Kupfersalzlösung enthaltenden Kapillarrohres vorgesehen ist, wobei für die Betriebsdauer die an der Innenwand sich abscheidende, dem fließenden Strom äquivalente Kupfermenge dient.

**Claims**

1. A process for the long-term monitoring of the emission and taking-up of noxious gaseous substances and of the moisture content of gases, in particular in air, in which for detection purposes, the chemical reaction or adsorption which takes place between the noxious substance or the moisture as reaction gas A with a stable reaction partner B is evaluated, for quantitative evaluation, the change in volume in the reaction partner B which is produced by the reaction gas A being converted into an

equivalent electrical value, characterised in that this change in volume is converted into an equivalent change in the pressure of the system, and this change in pressure is measured by measuring the electrical resistance of graphite particles which records the change in pressure, using a thin membrane of a piezoceramic material, or semi-conductor-based pressure sensors.

2. A process according to Claim 1, characterised in that the graphite particles are provided in granular form, spherical form, or as stacked discs.

3. A process according to one of Claims 1 and 2, characterised in that the reaction chamber is preceded by a filter layer which absorbs components likely to disturb the reaction or adsorption.

4. Apparatus for carrying out the process claimed in one of Claims 1 to 3, comprising a reactor consisting of a tubular reaction chamber (1) provided with a gas inlet (6) and a suction device (5), characterised in that, at that end of the reaction chamber (1) which lies opposite to the gas inlet (6), there is arranged a system which indicates the change in pressure and which consists of a stack of graphite discs (7) and an electrical resistance measuring arrangement, the terminals (8, 9) of which are connected to the uppermost and lovermost graphite discs (7).

5. Apparatus according to Claim 4, characterised in that the reaction chamber (1) is in the form of a curved tube.

6. Apparatus according to Claim 4, characterised in that the reaction chamber (1) is preceded by a filter layer in a chamber (3) which has a rigid wall (4) opposite to the reaction chamber (1).

7. Apparatus according to one of Claims 4 to 6, characterised in that, in place of the tubular reaction chamber (1), a flexible pipe is provided, which lies in the reactor axis and is filled with liquid, the change in pressure being recorded by means of liquid contained in the pipe and hydraulically transmitted to the measuring system.

8. Apparatus according to one of Claims 4 to 7, characterised in that an operating-time register, in the form of a capillary tube containing a copper salt solution, is provided, the amount of copper which is deposited on the inner walls and which is equivalent to the flow, serving to represent the operating time.

**Revendications**

1. Procédé de contrôle de longue durée des émissions et de la présence de substances nocives de forme gazeuse ainsi que du degré d'humidité de gaz, notamment dans l'air, qui consiste à utiliser, comme principe de détection, la réaction chimique ou l'adsorption de la substance nocive ou de l'humidité, servant de gaz de réaction A, sur un partenaire de réaction B solide et à l'exploiter quantitativement en transformant la variation de volume du partenaire de réaction B provoquée par le gaz de réaction A en une grandeur électrique équivalente, caractérisé en ce qu'il consiste à transformer cette variation de volume en une variation équivalente de pression du système et à mesurer cette variation de pression en mesurant la résistance électrique de particules de graphite indiquant la variation de pression, au moyen d'une membrane mince d'une céramique piézoélectrique ou par l'intermédiaire de capteurs de pression à base de semiconducteur.

2. Procédé suivant la revendication 1, caractérisé en ce que les particules de graphite sont sous forme de graviers, de billes, ou de pastilles empilées.

3. Procédé suivant l'une des revendications 1 et 2, caractérisé en ce qu'une couche filtrante absorbant les constituants gênant la réaction ou l'adsorption est montée en amont de la chambre de réaction.

4. Dispositif pour exécuter le procédé selon l'une des revendications 1 à 3, comprenant un réacteur constitué d'une chambre de réaction tubulaire (1) munie d'une ouverture d'entrée pour les gaz (6) et d'un dispositif d'aspiration (5), caractérisé en ce que, sur le côté de la chambre de réaction (1), opposé à l'ouverture d'entrée pour les gaz (6), est disposé un système indiquant la variation de pression et constitué d'une pile de pastilles de graphite (7) et d'un agencement de mesure à résistance électrique, dont les bornes (8, 9) sont reliées à la pastille de graphite (7) la plus élevée et à la pastille de graphite (7) la plus basse.

5. Dispositif suivant la revendication 4, caractérisé en ce que la chambre de réaction (1) est agencée en tube courbé.

6. Dispositif suivant la revendication 4, caractérisé en ce qu'en amont de la chambre de réaction (1) une couche filtrante est montée dans une chambre (3) présentant une paroi rigide (4) en face de la chambre de réaction (1).

7. Dispositif suivant l'une des revendications 4 à 6, caractérisé en ce qu'il est prévu, au lieu de la chambre de réaction (1) tubulaire, un conduit souple empli de liquide et disposé dans l'axe du réacteur, la variation de pression étant reçue par l'intermédiaire du liquide se trouvant dans le conduit souple, et étant transmise hydrauliquement au système de mesure.

8. Dispositif suivant l'une des revendications 4 à 7, caractérisé en ce qu'il est prévu un compteur d'heures de fonctionnement, sous forme d'un tube capillaire contenant une solution de sel de cuivre, la quantité de cuivre, équivalant au courant qui s'écoule et se déposant sur la paroi intérieure, servant à déterminer la durée de fonctionnement.

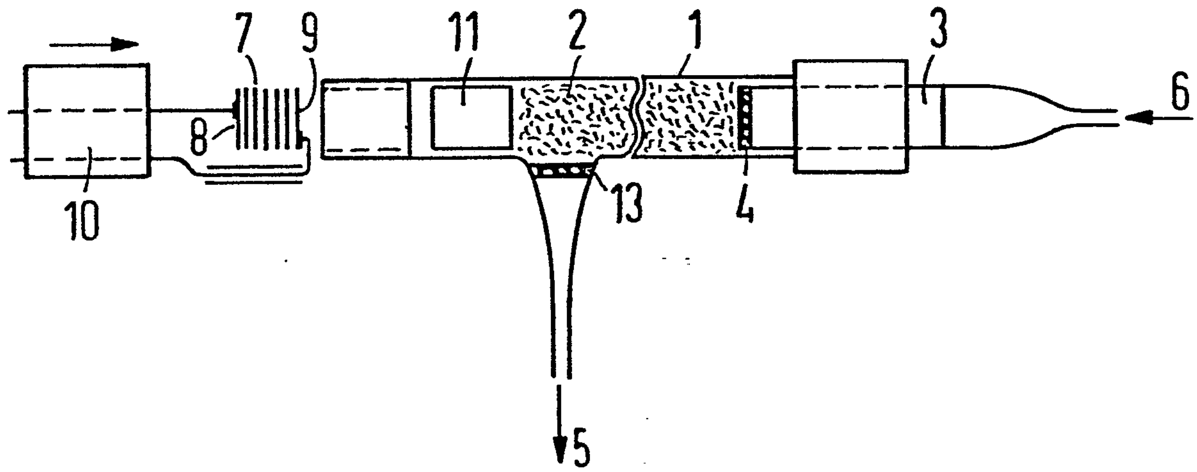
7
9
11
2
1
3
6
8
10
13
4
5